# EUROPÄISCHE PATENTSCHRIFT

(11) **EP 0 499 924 B1**
(45) Veröffentlichungstag und Bekanntmachung des Hinweises auf die Patenterteilung: **05.10.1994**
(21) Anmeldenummer: 92102154.9
(22) Anmeldetag: 10.02.1992
(51) Int. Cl.: C07C 68/04, C07C 68/06, C07C 69/96, C07D 317/36, C07D 317/38

(54) **Verfahren zur Herstellung von Dialkylcarbonaten**
Process for the preparation of dialkyle carbonates
Procédé de préparation de carbonates de dialkyle

(30) Priorität: 22.02.1991 DE 4105554
(43) Veröffentlichungstag der Anmeldung: 26.08.1992
(73) Patentinhaber: BAYER AG, 51368 Leverkusen (DE)
(72) Erfinder: Buysch, Hans-Josef, Dr., W-4150 Krefeld (DE); Klausener, Alexander, Dr., W-4150 Krefeld (DE)

(56) Entgegenhaltungen:
- EP-A- 0 001 777
- EP-A- 0 069 494
- EP-A- 0 119 840
- EP-A- 0 255 252

## Beschreibung

Die vorliegende Erfindung betrifft ein Verfahren zur Herstellung von Dialkylcarbonaten aus Alkylenoxiden, CO₂ und Alkanolen, worin in einer ersten Stufe zunächst das zugehörige Alkylencarbonat gebildet wird, das dann mit dem Alkanol umgeestert wird. Das Verfahren ist durch die Benutzung des weiter unten beschriebenen bifunktionellen Katalysators ausgezeichnet.

Es ist bekannt, Alkylenoxide mit CO₂ in Gegenwart von Katalysatoren zu Alkylencarbonaten umzusetzen. Dazu sind jedoch zur Erzielung von technisch ausreichenden Reaktionsgeschwindigkeiten hohe Temperaturen und Drücke erforderlich. Die Anwendung dieser Reaktionsbedingungen ist jedoch insofern problematisch, als einerseits besonders die niedermolekularen Alkylencarbonate technisches Interesse beanspruchen, und andererseits die als Ausgangsstoffe erforderlichen Alkylenoxide bei diesen Temperaturen und Drücken zur Zersetzung neigen und daher sicherheitstechnische Einrichtungen erfordern. Darüberhinaus ist allgemein ein großer apparativer Aufwand zur Beherschung der Temperaturen und Drücke erforderlich. Als Katalysatoren für die Durchführung der genannten Reaktion wurden beispielhaft bisher die folgenden genannt: Ammonium-, Phosphonium- oder Sulfoniumhalogenide (DE-OS 3 244 456); eine Kombination aus protischen Verbindungen, wie Alkoholen, und stickstoffhaltigen Basen (DE-OS 2 611 087); Arsoniumhalogenide (EP 180 387); Phosphoniumhalogenide (US 2.994.705); tertiäre Phosphine mit Alkoholen (WO 84/03 701); Alkalimetall-Transfer-Katalysatoren mit Kronenethern, Diazabicycloocten oder Tetramethylethylendiamin als Komplexliganden (Monatsh. Chem. 115 (1984), 205-214).

Es ist weiterhin bekannt, Alkylencarbonate mit Alkoholen zu Dialkylcarbonaten umzuestern. So werden gemäß DE-OS 2 740 243 bei der Reaktion von Ethylen- oder Propylencarbonat mit Methanol oder Ethanol in Gegenwart von Alkalimetallverbindungen hohe Ausbeuten an Dimethyl- bzw. Diethylcarbonat erhalten. In dieser Reaktion sind Temperaturen von etwa 150-200°C und die zugehörigen Drücke erforderlich. Bei niederen Temperaturen ergeben sich unbrauchbar lange Umesterungszeiten.

Auch im Verfahren der DE-OS 2 740 251, bei welchem in Gegenwart von Thalliumverbindungen gearbeitet wird, sind hohe Ausbeuten nur bei hohen Temperaturen und den zugehörigen Drücken erreichbar.

Weiterhin ist es bekannt, die Alkylcarbonate direkt aus Alkylenoxiden, CO₂ und Alkoholen zu gewinnen (EP 1777). Auch hierbei müssen hohe Temperaturen und zugehörige hohe Drücke angewandt werden. Außerdem werden als Nebenprodukte Glykolether erhalten, die in einer separaten Destillation abgetrennt werden müssen.

In Chem. Bar. 119 (1986), 1090-1094 wird vorgeschlagen, die Umsetzung eines Alkylenoxids (Oxirans) mit CO₂ bei Normaldruck und Raumtemperatur durchzuführen. Hierzu wird die Lösung eines Katalysators in einem Alkylenoxid mit CO₂ gesättigt. Verwendet man gemäß diesem Vorschlag bifunktionelle Katalysatoren, die aus als Lewis-Säuren wirkenden Metalldichloriden und Oniumiodiden bzw. -bromiden bestehen, so werden unter den angegebenen milden Bedingungen in einigen Fällen beträchtliche Ausbeuten an Alkylenoxiden erhalten. Die Reaktionszeiten sind jedoch im allgemeinen sehr lang. Neben diesem Nachteil der langen Reaktionszeit und der in den meisten Fällen ungenügenden Ausbeute ist es wegen des Sicherheitsrisikos im technischen Maßstab unmöglich, große Mengen an Alkylenoxid in Gegenwart von Katalysatoren zu handhaben. Dieser Vorschlag steht demnach aus mehreren Aspekten sehr im Gegensatz zu den vorher erwähnten Verfahren, in denen bei den genannten hohen Temperaturen und hohen Drücken vielfach Ausbeuten von etwa 99 % erhalten werden.

Zur Umesterung der gemäß den beschriebenen Verfahren erhaltenen Alkylencarbonaten mit Alkoholen müssen die in den Alkylencarbonaten erhaltenen Katalysatoren in der Regel abgetrennt werden, was vielfach durch Abdestillieren der Alkylencarbonate von den Katalysatorresten geschieht. Die verbliebenen Katalysatoren können in der Regel erneut verwendet werden. Einige der für die Alkylencarbonatbildung vorgeschlagenen Katalysatoren, wie beispielsweise die quartären Ammoniumsalze, sind auch als Umesterungskatalysatoren eingesetzt worden; die erzielten Geschwindigkeiten der Umesterung sind jedoch unzureichend.

Demnach gibt es bisher kein technisches Verfahren, das die Alkylencarbonatsynthese aus Alkylenoxid und Kohlendioxid unter technisch noch brauchbaren Bedingungen durchzuführen erlaubt, wobei gleichzeitig hohe bis praktisch qantitative Umsätze und ebenfalls praktisch qantitative Ausbeuten an Alkylencarbonaten erzielt werden, und bei dem eine direkte weitere Umsetzung zu den gewünschten Dialkylcarbonaten durch Umesterung mit Alkanolen erfolgt, wobei in bevorzugter Weise auf eine Isolierung und Zwischenreinigung der Alkylencarbonate verzichtet werden kann und bei dem auch in dieser zweiten (Umesterungs)Stufe hohe Umsätze und Selektivitäten bezüglich der letztendlich gewünschten Dialkylcarbonate erzielt werden.

Es wurde nun gefunden, daß man beide Reaktionsstufen, nämlich die Bildung des Alkylencarbonats und die nachfolgende Umesterung vorteilhaft in Gegenwart des weiter unten beschriebenen bifunktionellen Katalysators der Formel (I) durchführen kann, wobei man bei technisch geeigneten Temperaturen von 40-190°C und Drücken unterhalb von 10 bar arbeiten kann und wobei beide Reaktionsstufen durch das gleiche Katalysatorsystem erfindungsgemäß vorteilhaft beeinflußt werden.

Dies ist überraschend, weil gemäß Chem. Ber. 123 (1990), 277-283, die Reaktion von CO₂ mit Alkylenoxiden in Anwesenheit von bifunktionellen Katalysatoren schon bei Temperaturen oberhalb von 50°C rückläufig wird und damit die Spaltung der Alkylencarbonate in CO₂, Alkylenoxide und Aldehyde beginnt (loc. cit. S. 278, linke Spalte, 2. Absatz).

Das erfindungsgemäße Verfahren ist weiterhin dadurch überraschend, daß die Umesterung der intemediär gebildeten Alkylencarbonate mit Alkoholen in Gegenwart der gleichen bifunktionellen Katalysatoren der Formel (I) schon bei ebenfalls technisch brauchbaren milden Bedingungen schnell und glatt verläuft, während die Einzelbestandteile dieser bifunktionellen Katalysatoren die Umesterung nur langsam und damit mit technisch unbefriedigender Geschwindigkeit ermöglichen. Selbst die Gemische der Einzelkomponenten der bifunktionellen Katalysatoren wirken in der Umesterungstufe ungenügend, wenn sie nicht vorher zur Bildung der Alkylencarbonate erfindungsgemäß eingesetzt worden waren. Die bifunktionellen Katalysatoren der Formel (I) erfahren demnach durch ihren Einsatz in der ersten Stufe des erfindungsgemäßen Verfahrens eine überraschende Steigerung ihrer katalytischen Aktivität für die Umesterung gemäß zweiter Stufe des erfindungsgemäßen Verfahrens.

Die Erfindung betrifft demnach ein Verfahren zur Herstellung von Dialkylcarbonaten aus Alkylenoxiden, CO₂ und Alkanolen in Gegenwart eines Katalysators, das dadurch gekennzeichnet ist, daß man in einer ersten Stufe ein Alkylenoxid mit 2-8 C-Atomen mit CO₂ in dem zunächst zu bildenden Alkylencarbonat als Reaktionsmedium bei 40-190°C, bevorzugt bei 50-170°C, besonders bevorzugt bei 60-160°C und einem Druck unterhalb von 10 bar, bevorzugt unterhalb von 8 bar, besonders bevorzugt unterhalb von 5 bar und ganz besonders bevorzugt unterhalb von 3 bar umsetzt und in einer zweiten Stufe das so gebildete Alkylencarbonat mit einer (cyclo)aliphatischen C₁-C₁₀-Monohydroxylverbindung, die durch C₃-C₆-Cycloalkyl oder Phenyl substituiert sein kann, bei 50-160°C, bevorzugt bei 60-150°C, besonders bevorzugt bei 70-140°C und unter dem Eigendruck umestert, wobei in beiden Stufen in Gegenwart eines bifunktionellen Katalysators der Formel

[Aₐ-X_{b}]ₘ . [B_{c}Y_{d}]ₙ (I)

gearbeitet wird, worin
- A: das Kation eines Metalles ist, das der
dritten Periode und Gruppe IIa, der
vierten Periode und Gruppe IIa, IVa - VIIIa, Ib oder II b, der
fünften Periode und Gruppe IIa, IVa - VIIa, IIb oder IVb bzw. der
sechsten Periode und Gruppe IIa - VIa des Periodensytems der Elemente in der Kurzperiodenform angehört,
- X: das Anion einer anorganischen oder organischen Säure ist,
- B: für ein Kation aus der Gruppe der Alkali- oder Erdalkalikationen, der quaternären Ammonium-, Phosphonium-, Arsonium- oder Stiboniumionen und der ternären Sulfoniumkationen steht,
- Y: ein Halogenidion, bevorzugt Bromid oder Iodid, besonders bevorzugt Iodid, ist, wobei X und Y ihre Stellung tauschen können, wenn wenigstens eines der Anionen Bromid oder Iodid ist,
- a und b: unabhängig voneinander ganze Zahlen von 1 bis 5 und
- c und d: unabhängig voneinande ganze Zahlen von 1 bis 3 darstellen, wobei den Forderungen der Valenzen der Kationen und Anionen zur Bildung eines neutralen Salzes zu entsprechen ist und
- m und n: unabhängig voneinander Werte von 0,001 - 1 annehmen.

Das erfindungsgemäße Verfahren sei an der Bildung des Dimethylcarbonats aus Ethylenoxid, CO₂ und Methanol durch die folgende Formelgleichung beschrieben: 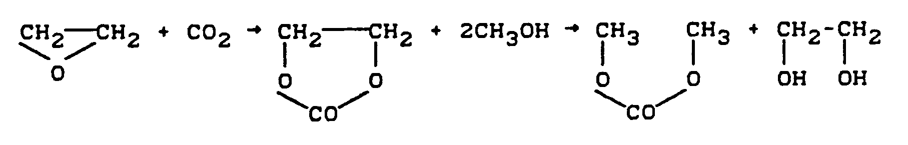
Alkylenoxide mit 2-8 C-Atomen sind in bevorzugter Weise solche der Formel: 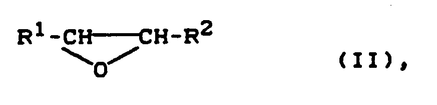
worin
- R¹: Wasserstoff, geradkettiges oder verzweigtes C₁-C₆-Alkyl, geradkettiges oder verzweigtes C₂-C₆-Alkenyl oder Chlor bedeutet und
- R²: Wasserstoff oder C₁-C₃-Alkyl bedeutet,
wobei R¹ und R² gemeinsam auch Trimethylen, Tetramethylen oder Pentamethylen darstellen können und die Gesamtzahl der C-Atome 8 nicht übersteigt.

In besonders bevorzugter Weise werden als Alkylenoxid Ethylenoxid, Propylenoxid, 1,2- oder 2,3-Butylenoxid, Cyclohexenoxid, Vinyloxiran oder Epichlorhydrin, in besonders bevorzugter Weise Ethylenoxid oder Propylenoxid, eingesetzt.

Als (cyclo)aliphatische C₁-C₁₀-Monohydroxylverbindung, die durch C₃-C₆-Cycloalkyl oder Phenyl substituiert sein kann, sei ein geradkettiger oder verzweigter C₁-C₁₀-Alkanol, oder ein C₃-C₈-Cycloalkanol genannt. In beiden Fällen kann eine Substitution durch C₃-C₆-Cycloalkyl oder Phenyl vorliegen. Beispiele für solche Monohydroxylverbindungen sind: Methanol, Ethanol, Propanol, Isopropanol, Butanol, Isobutanol, Amylalkohol, Hexylalkanol, Octylalkanol, Decylalkanol, Cyclopropylalkanol, Cyclopentylalkanol, Cyclohexanol, Methyl- und Dimethyl-cyclohexanol, Cyclohexan-methanol, Benzylalkohol; in bevorzugter Weise seien geradkettiges oder verzweigtes C₁-C₄-Alkanol der genannten Art, besonders bevorzugt Methanol und Ethanol, genannt.

Das erfindungsgemäß einzusetzende CO₂ kann mit inerten Gasen, wie Stickstoff, Wasserstoff, Kohlenmonoxid, niederen Kohlenwasserstoffen, verunreinigt sein und kann aus natürlichen Quellen oder aus industriellen Abgasen stammen.

Das Molverhältnis der Reaktionspartner Alkylenoxid und CO₂ im ersten Reaktionsschritt beträgt in der Regel etwa 1:1. Man kann jedoch auch einen Überschuß an CO₂ verwenden, der grundsätzlich nicht kritisch ist, aus okönomischen Erwägungen jedoch begrenzt werden soll und selten mehr als das doppelte der benötigten Menge beträgt.

Bei der Umesterungsreaktion (2. Verfahrensschritt) ist das Molverhältnis von Alkohol zu vorhandenem Alkylencarbonat in weiten Grenzen variabel und kann von 1:1 bis 1:100, bevorzugt von 1:2 bis 1:50, besonders bevorzugt 1:2 bis 1:30, ganz besonders bevorzugt von 1:2 bis 1:20 betragen. Aus ökonomischen Gründen wird jedoch ein Verhältnis von 1:1 bis 1:20 bevorzugt.

Das erfindungsgemäße Verfahren ist besonders durch den Einsatz der bifunktionellen Katalysatoren der Formel (I) gekennzeichnet. In diesen bifunktionellen Katalysatoren wird das molare Verhältnis der beiden in eckigen Klammern stehenden Komponenten durch die Indices m und n ausgedrückt. Diese Indices können unabhängig voneinander Werte von 0,001 bis 1, bevorzugt von 0,01 bis 1, besonders bevorzugt von 0,05 bis 1 und ganz besonders bevorzugt von 0,1 bis 1 annehmen. Innerhalb der eckigen Klammern stehen neutrale Salze aus je einem Kation und einem Anion. Die Indices a und b stellen unabhängig voneinander ganze Zahlen von 1 bis 5 dar; die Indices c und d unabhängig voneinander ganze Zahlen von 1 bis 3, wobei den Forderungen der Valenzen der Kationen und Anionen zur Bildung solcher neutraler Salze zu entsprechen ist.
- A: ist das Kation eines Metalles, das der
dritten Periode und Gruppe IIa, der
vierten Periode und Gruppe IIa, IVa - VIIIa, Ib oder IIb, der
fünften Periode und Gruppe IIa, IVa - VIIa, IIb oder IVb bzw. der
sechsten Periode und Gruppe IIa - VIa des Periodensytems der Elemente in der Kurzperiodenform an.

Die in Frage kommenden Metalle für das Kation A entnimmt der Fachmann den üblichen Darstellungen des Periodensystems der Elemente (Mendelejew). In bevorzugter Weise handelt es sich bei A um das Kation eines der Metalle Mg, Ca, Sr, Ba, Zn, Cu, Mn, Co, Ni, Fe, Cr, Mo, W, Ti, Zr, Sn, Hf, V und Ta, in besonders bevorzugter Weise um das Kation eines der Metalle Mg, Ca, Zn, Co, Ni, Mn, Cu und Sn. Außer den nicht komplexierten Kationen der genannten Metalle kommen auch kationische Oxokomplexe der genannten Metalle in Frage, wie beispielsweise Titanyl TiO⁺⁺ und Chromyl CrO₂⁺⁺.

Das zum Kation A gehörige Anion X ist das einer anorganischen oder organischen Säure. Eine solche anorganische oder organische Säure kann einbasisch oder zweibasisch oder dreibasisch sein. Solche Säuren und ihre Anionen sind dem Fachmann bekannt. Beispiele für Anionen einbasischer anorganischer oder organischer Säuren sind: Fluorid, Bromid, Chlorid, Iodid, Nitrat, das Anion einer Alkancarbonsäure mit 1-16 C-Atomen und Benzoat; Beispiele für Anionen zweibasischer anorganischer oder organischer Säuren sind: Sulfat, Oxalat, Succinat, Fumarat, Maleinat, Phthalat und weitere; Beispiele für dreibasische anorganische oder organische Anionen sind: Phosphat oder Citrat. Bevorzugte Anionen X im Katalysator der Formel (I) sind: Fluorid, Chlorid, Bromid, Iodid, Sulfat, Nitrat, Phosphat, Formiat, Acetat, Propionat, Oxalat, Butyrat, Citrat, Succinat, Fumarat, Maleinat, Benzoat, Phthalat, Decanoat, Stearat, Palmitat und Laurinat. Besonders bevorzugte Anionen X sind: Chlorid, Bromid, Iodid, Acetat, Laurinat, Stearat, Palmitat, Decanoat, Nitrat oder Sulfat.

Als Kation B in den Katalysatoren der Formel (I) kommt eines aus der Gruppe der Alkali- oder Erdalkalikationen, der quaternären Ammonium-, Phosphonium-, Arsonium- oder Stibonium-Kationen und der ternären Sulfonium-Kationen in Frage.

Als (Erd)Alkalimetallkationen seien genannt: das Lithium-, Natrium-, Kalium-, Rubidium- Cäsium-, Magnesium-, Calcium-, Strontium- und Bariumkation, bevorzugt die genannten Alkalimetallkationen, besonders bevorzugt das Natrium- und das Kaliumkation.

In bevorzugter Weise kommen als Kation B solche der Formeln in Frage 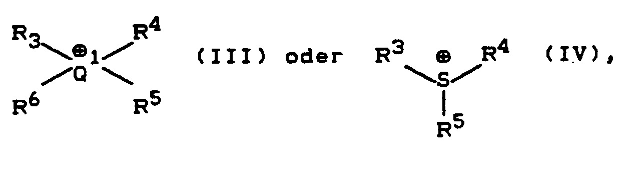
worin
- Q¹: für N, P, As oder Sb steht und
- R³, R⁴, R⁵ und R⁶: unabhängig voneinander geradkettiges oder verzweigtes C₁-C₁₈-Alkyl oder C₇-C₁₂-Aralkyl sind und einer der Reste R³ bis R⁶ auch C₆-C₁₂-Aryl sein kann.

In besonders bevorzugter Weise ist B ein Kation der Formel 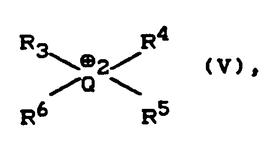
worin
- Q²: für N oder P, bevorzugt für N steht.

In ganz besonders bevorzugter Weise treten im Rahmen der Formel (III) bzw. (V) an die Stelle der Reste R³, R⁴, R⁵ bzw. R⁶ die Reste R¹³, R¹⁴, R¹⁵ bzw. R¹⁶, die unabhängig voneinander geradkettiges oder verzweigtes C₁-C₁₂-Alkyl oder C₇-C₈-Aralkyl bedeuten und einer der Reste R¹³ bis R¹⁶ auch Phenyl sein kann. In weiterhin ganz besonders bevorzugter Weise treten an die Stelle der Reste R¹³, R¹⁴, R¹⁵ bzw. R¹⁶ die Reste R²³, R²⁴, R²⁵ bzw. R²⁶, die unabhängig voneinander C₁-C₈-Alkyl oder Benzyl bedeuten und einer der Reste R²³ bis R²⁶ auch Phenyl sein kann.

Geradkettiges oder verzweigtes C₁-C₁₈-Alkyl ist beispielsweise Methyl, Ethyl, Propyl, Isopropyl, Butyl, Isobutyl, Hexyl, Octyl, Dodecyl, Hexadecyl oder Octadecyl.

C₇-C₁₂-Aralkyl ist beispielsweise Benzyl, Phenylethyl, Phenylpropyl, Naphthyl-methyl oder Naphthyl-ethyl; bevorzugtes Aralkyl ist Benzyl oder Phenylethyl, ganz besonders bevorzugtes Aralkyl ist Benzyl.

Bevorzugtes Alkyl hat 1-12 C-Atome, besonders bevorzugtes Alkyl hat 1-8 C-Atome.

Das Anion Y im Katalysator der Formel (I) ist ein Halogenidion, wie Fluorid, Chlorid, Bromid oder Iodid, bevorzugt Bromid oder Iodid, besonders bevorzugt Iodid. Es kann jedoch auch die Bedeutung von anderen, unter X genannten Anionen besitzen, wenn im konkreten Fall das Anion X Bromid oder Iodid ist.

Der bifunktionelle Katalysator der Formel (I) wird in einer Menge von 0,005-10 Gew.-%, bevorzugt 0,01-5 Gew.-%, besonders bevorzugt 0,02-3 Gew.-%, bezogen auf das vorgelegte Alkylencarbonat, in der ersten Stufe eingesetzt. Er wird in Menge von 0,005-5 Gew.-%, bevorzugt 0,01-3 Gew.-%, besonders bevorzugt 0,01-1 Gew.-%, bezogen auf das gesamte Umesterungsgemisch, in der zweiten Stufe eingesetzt.

Die Reaktionstemperatur liegt in der ersten Stufe bei 40-190°C, bevorzugt 50-170°C, besonders bevorzugt 60-160°C und in der zweiten Stufe bei 50-160°C, bevorzugt 60-150°C, besonders bevorzugt 70-140°C.

Die erste Stufe wird bei einem Druck von unterhalb 10 bar, bevorzugt unterhalb 8 bar, besonders bevorzugt unterhalb von 5 bar und ganz besonders bevorzugt unterhalb von 3 bar durchgeführt. Die Untergrenze des Druckes für die erste Reaktionsstufe liegt bei etwa 1 bar. Die zweite Stufe wird im allgemeinen unter dem sich einstellenden Eigendruck durchgeführt, der im wesentlichen durch den Siedepunkt des verwendeten Umesterungalkohols unter den jeweiligen Bedingungen bestimmt wird. Die Einstellung eines höheren Druckes bringt keine weiteren Vorteile, ist jedoch auch unschädlich für die Durchführung des erfindungsgemäßen Verfahrens.

Die Reaktion der ersten Stufe des erfindungsgemäßen Verfahrens wird in dem jeweiligen Alkylenglykolcarbonat durchgeführt, das bei dieser Reaktionsstufe entsteht, also in Ethylenglykolcarbonat, wenn Ethylenoxid mit CO₂ umgesetzt wird, und in Propylenglykolcarbonat, wenn Propylenoxid mit CO₂ umgesetzt wird. Die Menge an dem jeweiligen Alkylencarbonat ist so zu bemessen, daß eine zügige Absorption und Umsetzung der Reaktionskomponenten gewährleistet ist. Diese Menge kann durch Vorversuche ermittelt werden und beträgt im allgemeinen mindestens die Menge der stündlich zu produzierenden Menge an Alkylencarbonat im Reaktionsgefäß.

Die zweite Reaktionsstufe kann grundsätzlich so durchgeführt werden, daß das gebildete Alkylencarbonat abgetrennt und gereinigt wird und sodann in Gegenwart eines Katalysators der Formel (I) mit dem Alkohol umgesetzt wird, der als Esterkomponente gewünscht wird. Der Katalysator der Formel (I) für die zweite Reaktionsstufe kann der aus der ersten Reaktionsstufe oder ein anderer sein, der unter die Formel (I) fällt und der ebenfalls bereits einmal für die Reaktion der ersten Stufe Verwendung gefunden hatte. In bevorzugter Weise wird jedoch das Reaktionsgemisch der ersten Stufe nicht besonders aufgearbeitet oder gereinigt, sondern sofort unter Hinzufügung der (cyclo)aliphatischen C₁-C₁₀-Monohydroxylverbindung der Umesterung zugeführt.

Die Reaktion der ersten Stufe kann in üblichen Reaktionsgefäßen durchgeführt werden, die für den angegegebenen Druck ausgelegt sind und eine Rühreinrichtung besitzen. Die Dosierung der Reaktionskomponenten erfolgt durch Eindüsung oder Einpumpen in der dem Fachmann geläufigen Weise. Das CO₂ und, soweit die Alkylenoxide verdampfbar sind, deren Dämpfe werden bevorzugt mit Hilfe von Begasungsrührern, Fritten oder ähnlichen Einrichtungen dosiert, die eine rasche und gleichmäßige Verteilung dieser Reaktionspartner gewährleisten, wodurch wiederum Absorption und rasche Umsetzung gewährleistet sind. Bei diskontinuierlicher Reaktionsführung kann beispielsweise eine Menge Alkylencarbonat und die oben beschriebene Menge an bifunktionellem Katalysator vorgelegt werden und sodann durch Zufügen von Alkylenoxid und CO₂ die Reaktion soweit fortgesetzt werden, bis das Reaktionsgefäß gefüllt ist. Für eine halbkontinuierliche oder kontinuierliche Reaktionsführung kann man jedoch auch aus dem Reaktionsgefäß entsprechend der neu gebildeten Menge an Alkylencarbonat Reaktionsgemisch abziehen, wobei dieser Abzug kontinuierlich oder absatzweise erfolgt. Bei einer solchen Reaktionsvariante wird dem Reaktionsgefäß in entsprechender Weise bifunktioneller Katalysator der Formel (I) zugesetzt, damit die Reaktionsmischung nicht an Katalysator verarmt. In entsprechender Weise ist es weiterhin möglich, den Abzug des neugebildeten Alkylencarbonats erst aus einem zweiten oder weiteren Reaktor vorzunehmen, in welchem eine Nachreaktion stattgefunden hat.

Eine weitere, besonders bevorzugte Ausführungsform der Alkylencarbonatsynthese (erste Stufe des erfindungsgemäßen Verfahrens) bedient sich der Blasensäule als Reaktionsgefäß, in die von unten die gasförmigen Komponenten über eine Gasverteilung eingedüst werden, wie oben beschrieben, und flüssige Komponenten über übliche Dosierorgane. Man erhält auf diese Weise in einfachen Reaktionsgefäßen eine rasche und vollständige Umsetzung der Reaktionspartner. Eine solche Blasensäule ist ein typisches Reaktionsgefäß für die Durchführung in kontinuierlicher Arbeitsweise.

Die Umesterung kann ebenso in üblichen Reaktoren, wie Rührbehältern, Autoklaven, Rohrreaktoren und Rohrbündelreaktoren erfolgen, die für die kontinuierliche Arbeitsweise auch zu Kaskaden zusammengestellt werden können. Bevorzugt bei kontinuierlicher Arbeitsweise ist eine Durchführung der Umesterung in Rohrreaktoren, in denen eine Rückvermischung weitgehend unterbunden werden kann, damit am Ende des Reaktors das Umesterungsgleichgewicht eingestellt und eine größtmögliche Umesterungsrate erzielt wird. Eine weitere Möglichkeit der Umesterung bietet die Rieselphase, bei der Alkylcarbonat über Füllkörper in einer Kolonne rieselnd dem Strom von Oxiran und CO₂ entgegengeführt wird.

Die Aufarbeitung des bei der Umesterung erhaltenen Gemisches erfolgt in bevorzugter Weise destillativ. Hierbei können fraktioniert etwa überschüssige (cyclo)aliphatische Monohydroxylverbindung und das erhaltende Carbonat dieser Monohydroxylverbindung erhalten werden. Der als Rückstand verbleibende Katalysator kann in bevorzugter Weise in die erste Reaktionsstufe zurückgeführt werden. Für den Fall, daß zur Umesterung eine unterstöchiometrische Menge an Monohydroxylverbindung eingesetzt worden war, bleibt nach der destillativen Aufarbeitung der Katalysator in überschüssigem Alkylencarbonat zurück. Auch bei dieser Arbeitsweise kann der Katalysator in die erste Stufe zurückgeführt werden, wobei das gleichzeitig vorhandene Alkylencarbonat das erfindungsgemäß zu benutzende Reaktionsmedium darstellt.

### Beispiel 1 -12

Ein 500 ml-Dreihalskolben mit Rührer, Thermometer und Gaseinleitungsrohr wurde mit 200 g Ethylenglykolcarbonat und mit Katalysator gemäß folgender Tabelle beschickt, auf 50° erhitzt und unter Rühren mit einem schwachen Strom von Ethylenoxid und Kohlendioxid begast. Nach jeweils 4 h wurde der Kolbeninhalt gewogen und die Gewichtszunahme bestimmt. Die gaschromatographische Analyse des Reaktionsproduktes ergab in jedem Fall, daß während der Reaktion keine Nebenprodukte gebildet wurden.

**Tabelle**

| (Beispiele 1-12) | | | |
|---|---|---|---|
| Nr. | Kat. | | Gew.-Zunahme g |
| | 1,4 g | 0,5 g | |
| 1 | NBu₄J¹⁾ | ZnCl₂ | 13 |
| 2 | " | MgCl₂ | 11 |
| 3 | " | CaCl₂ | 9 |
| 4 | " | CuCl₂ | 12 |
| 5 | " | Cu(OAc)₂²⁾ | 11 |
| 6 | " | SnCl₂ | 11 |
| 7 | " | NiCl₂ | 10 |
| 8 | " | CoCl₂ | 19 |
| 9 | " | Sn(OLau)₂³⁾ | 10 |
| 10 | " | Zn(OAc)₂ | 9 |
| 11 | KJ | ZnCl₂ | 12 |
| 12 | ⁴) | ⁴) | 13 |

| | | | |
|---|---|---|---|
| ¹) NBu₄J = Tetrabutylammoniumiodid | | | |
| ²) OAc = Acetat | | | |
| ³) OLau = Laurinat | | | |
| ⁴) Katalysator = Rückstand aus Beispiel 1 nach Destillation des bei der nachfolgenden Umesterung erhaltenen Reaktionsgemisches | | | |

### Beispiel 13

In einem 500 ml-Dreihalskolben mit Rührer, Thermometer und Gaseinleitungsrohr wurden 330 g Ethylenglykolcarbonat, worin 5 g NBu₄J und 1,25 g ZnCl₂ gelöst waren, bei 50° unter kräftigem Rühren während 6 Stunden mit insgesamt je 1 Mol Ethylenoxid und Kohlendioxid begast. Die Gewichtszunahme betrug 60 g. Nebenprodukte wurden nicht nachgewiesen.

### Beispiel 14

In ein senkrecht stehendes Rohr von 130 cm Länge und 3,0 cm Durchmesser, das mit einem Heizmantel und am Boden mit einer Fritte versehen war, wurden 700 g Ethylenglykolcarbonat, die 10 g Tetrabutylammmoniumiodid und 2,5 g Zinkchlorid enthielten, auf 80° erwärmt und durch die Fritte während 6 h mit insgesamt 70 g Kohlendioxid und 70 g Ethylenoxid begast. Das Gasgemisch wurde in der Blasensäule fast vollständig in Glykolcarbonat umgewandelt. Die Gewichtszunahme betrug demnach 139 g.

### Beispiel 15

Beispiel 14 wurde wiederholt, wobei statt 10 g Tetrabutylammoniumiodid 5 g und statt 2,5 g Zinkchlorid 1,25 g eingesetzt wurden und das Reaktionsgemisch auf 100° gehalten wurde. In 3 1/2 h wurden insgesamt je 50 g Kohlendioxid und Ethylenoxid durch die Fritte eingeleitet. Die Absorption des Gasgemisches und damit der Umsatz und die Umwandlung zu Ethylenglykolcarbonat waren praktisch vollständig. Die Gewichtszunahme am Ende der Reaktion betrug 99 g.

### Vergleichsbeispiel 1

Beispiel 14 wurde wiederholt, wobei statt des Katalysatorgemisches aus Tetrabutylammoniumiodid und Zinkchlorid lediglich 5 g Tetrabutylammoniumiodid verwendet wurde. Es wurde mit einem Gasgemisch aus 100 g Kohlendioxid und 100 g Ethylenoxid begast. Die Gewichtszunahme betrug 13 g.

### Vergleichsbeispiel 2

Beispiel 14 wurde wiederholt, wobei statt des Katalysatorgemisches aus Tetrabutylammoniumiodid und Zinkchlorid lediglich 5 g Zinkchlorid verwendet wurde. Es wurde mit einem Gasgemisch aus 100 g Kohlendioxid und 100 g Ethylenoxid begast. Die Gewichtszunahme betrug 12 g.

### Beispiel 15a

Beispiel 14 wurde wiederholt, wobei als Katalysator 2,5 g Kaliumiodid und 0,65 g Zinkchlorid eingesetzt wurden. Die Begasung erfolgte bei 80° während 4 h mit insgesamt 170 g Kohlendioxid und 120 g Ethylenoxid. Die Gewichtszunahme betrug 130 g. Wiederholte man diesen Versuch bei 95°, so betrug die Gewichtszunahme während 4 h 151 g.

### Beispiel 15b

Beispiel 14 wurde wiederholt, wobei als Katalysator 2,5 g Kaliumiodid und 0,19 g Zinkchlorid eingesetzt wurden. Die Begasung erfolgte bei 150°C. Nach 4 h betrug die Gewichtszunahme an Ethylenglykolcarbonat 183 g.

### Beispiel 16

Ein Gemisch aus 21 g des Produktes aus Beispiel 14, das noch den Katalysator enthielt, und 79 g Methanol wurden bei 60° gerührt. Die Umesterung des Gemisches wurde gaschromatographtisch verfolgt:

| Zeit (h) | Dimethylcarbonat (Gew.-%) | Ethylenglykolcarbonat (Gew.-%) |
|---|---|---|
| 0 | 0 | 21,2 |
| 3 | 5,1 | 10,2 |
| 6 | 8,2 | 7,5 |

Auch ohne Erwärmen des obigen Gemisches fand man bereits nach 24 h bei Raumtemperatur eine weitgehende Umesterung des Glykolcarbonates zum Dimethylcarbonat:

| Zeit (h) | Dimethylcarbonat (Gew.-%) | Ethylenglykolcarbonat (Gew.-%) |
|---|---|---|
| 0 | 0 | 21,2 |
| 24 | 11,6 | 5,8 |

### Vergleichsbeispiel 3-5

Wie in Beispiel 16 wurden 21 g reines Ethylenglykolcarbonat, jedoch ohne Katalysatorgehalt, und 79 g Methanol mit der entsprechenden Menge frischem Katalysator versetzt, 6 h auf 60°C gehalten und analysiert:

| Vergl. beispiel | NBu₄J (g) | ZnCl₂ (g) | Dimethylcarbonat (Gew.-%) | Ethylenglykolcarbonat (Gew.-%) |
|---|---|---|---|---|
| 3 | 0,25 | | 3,9 | 16,4 |
| 4 | | 0,06 | 3,4 | 16,9 |
| 5 | 0,25 | 0,06 | 3,1 | 17,1 |

Diese Versuche zeigen, daß die Katalysatoren, die nicht an der Ethylenglykolcarbonat-Synthese beteiligt waren, sondern frisch in die Umesterung eingesetzt werden, wesentlich weniger wirksam sind. In Beispiel 16 wurde mit derselben Katalysatormenge wie in Vergleichsbeispiel 5 (entsprechend Umrechnung aus Beispiel 14) ein mehr als doppelt so hoher Umesterungsgrad erzielt.

### Beispiel 17

Ein Gemisch von 21 g aus Beispiel 2 und 79 g Methanol wurden 2 h auf 80° bei etwa 2 bar Eigendruck erhitzt. Das Gemisch enthielt danach 15,4 Gew.-% Dimethylcarbonat und nur noch 2,6 Gew.-% Glykolcarbonat.
Führte man die Umesterung bei 140°C durch, so erhielt man das gleiche Ergebnis.

### Beispiele 18-22

Wiederholte man das Beispiel 17 mit weiteren Produkten aus den Beispielen 3, 4, 7 und 8, so erhielt man in jedem Fall im Reaktionsgemisch 15,2 bis 16,4 Gew.-% Dimethylcarbonat und 3,3 bis 2,2 Gew.-% Glykolcarbonat.

### Beispiel 23

765 g Methanol und 100 g aus Beispiel 15b wurden 2 h auf 150°C erhitzt. Man erhielt ein Reaktionsprodukt mit einem Gehalt von 9,8 % Dimethylcarbonat, 2,1 % Glykolcarbonat und 7,6 % Ethylenglykol.

### Beispiel 24

Zu 765 g Methanol, das im Autoklaven auf 150°C unter dem Eigendruck erhitzt wurde, drückte man rasch 100 g des Reaktionsgemisches aus Beispiel 14, das den Katalysator noch enthielt, und nahm vom Zeitpunkt der Zugabe an Proben zur Verfolgung der Umesterung. Spätestens nach 40 min. änderte sich die Zusammensetzung des Reaktionsproduktes nicht mehr und es bestand zu 80,5 % aus Methanol, 10,3 % Dimethylcarbonat, 1,3 % Glykolcarbonat und 7,9 % Ethylenglykol.

## Patentansprüche

1. Verfahren zur Herstellung von Dialkylcarbonaten aus Alkylenoxiden, CO₂ und Alkanolen in Gegenwart eines Katalysators, dadurch gekennzeichnet, daß man in einer ersten Stufe ein Alkylenoxid mit 2-8 C-Atomen mit CO₂ in dem zunächst zu bildenden Alkylencarbonat als Reaktionsmedium bei 40-190°C, bevorzugt bei 50-170°C, besonders bevorzugt bei 60-160°C und einem Druck unterhalb von 10 bar, bevorzugt unterhalb von 8 bar, besonders bevorzugt unterhalb von 5 bar, ganz besonders bevorzugt unterhalb von 3 bar umsetzt und in einer zweiten Stufe das so gebildete Alkylencarbonat mit einer (cyclo)aliphatischen C₁-C₁₀-Monohydroxylverbindung, die durch C₃-C₆-Cycloalkyl oder Phenyl substituiert sein kann, bei 50-160°C, bevorzugt bei 60-150°C, besonders bevorzugt bei 70-140°C und unter dem Eigendruck umestert, wobei in beiden Stufen in Gegenwart eines bifunktionellen Katalysators der Formel
[Aₐ-X_{b}]ₘ . [B_{c}Y_{d}]ₙ
gearbeitet wird, worin
A das Kation eines Metalles ist, das der
dritten Periode und Gruppe IIa, der
vierten Periode und Gruppe IIa, IVa - VIIIa, Ib oder IIb, der
fünften Periode und Gruppe IIa, IVa - VIIa, IIb oder IVb bzw. der
sechsten Periode und Gruppe IIa - VIa des Periodensytems der Elemente in der Kurzperiodenform angehört,
X das Anion einer anorganischen oder organischen Säure ist,
B für ein Kation aus der Gruppe der Alkali- oder Erdalkalikationen, der quaternären Ammonium-, Phosphonium-, Arsonium- oder Stiboniumkationen und der ternären Sulfoniumkationen steht,
Y ein Halogenidion, bevorzugt Bromid oder Iodid, besonders bevorzugt Iodid, ist, wobei X und Y ihre Stellung tauschen können, wenn wenigstens eines der Anionen Bromid oder Iodid darstellt,
a und b unabhängig voneinander ganze Zahlen von 1 bis 5 und
c und d unabhängig voneinander ganze Zahlen von 1 bis 3 darstellen, wobei den Forderungen der Valenzen der Kationen und Anionen zur Bildung eines neutralen Salzes zu entsprechen ist, und
m und n unabhängig voneinander Werte von 0,001 bis 1 annehmen.

2. Verfahren nach Anspruch 1, dadurch gekennzeichnet, daß ein Alkylenoxid der Formel 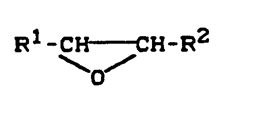 eingesetzt wird, worin
R¹ Wasserstoff, geradkettiges oder verzweigtes C₁-C₆-Alkyl, geradkettiges oder verzweigtes C₂-C₆-Alkenyl oder Chlor bedeutet und
R² Wasserstoff oder C₁-C₃-Alkyl bedeutet,
wobei R¹ und R² gemeinsam auch Trimethylen, Tetramethylen oder Pentamethylen darstellen können und die Gesamtzahl der C-Atome 8 nicht übersteigt.

3. Verfahren nach Anspruch 2, dadurch gekennzeichnet, daß als Alkylenoxid Ethylenoxid, Propylenoxid, 1,2- oder 2,3-Butylenoxid, Cyclohexenoxid, Vinyloxiran oder Epichlorhydrin, bevorzugt Ethylenoxid oder Propylenoxid, eingesetzt wird.

4. Verfahren nach Anspruch 1, dadurch gekennzeichnet, daß A das Kation eines der Metalle Mg, Ca, Sr, Ba, Zn, Cu, Mn, Co, Ni, Fe, Cr, Mo, W, Ti, Zr, Sn, Hf, V und Ta, bevorzugt eines der Metalle Mg, Ca, Zn, Co, Ni, Mn, Cu und Sn ist.

5. Verfahren nach Anspruch 1, dadurch gekennzeichnet, daß B ein Kation der Formel 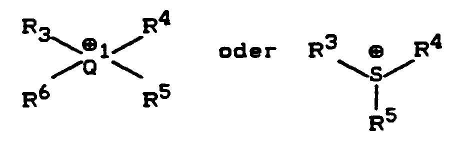 ist, worin
Q¹ für N, P, As oder Sb steht und
R³, R⁴, R⁵ und R⁶ unabhängig voneinander geradkettiges oder verzweigtes C₁-C₁₈-Alkyl oder C₇-C₁₅-Aralkyl sind und einer der Reste R³ bis R⁶ auch C₆-C₁₂-Aryl sein kann,
daß B bevorzugt ein Kation der Formel 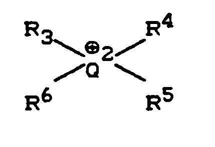 ist, worin
Q² für N oder P, bevorzugt für N steht.

6. Verfahren nach Anspruch 5, dadurch gekennzeichnet, daß an die Stelle von R³, R⁴, R⁵ bzw. R⁶ die Reste R¹³, R¹⁴, R¹⁵ bzw. R¹⁶ treten, die unabhängig voneinander geradkettiges oder verzweigtes C₁-C₁₂-Alkyl oder C₇-C₈-Aralkyl bedeuten und einer der Reste R¹³ - R¹⁶ auch Phenyl sein kann, daß bevorzugt an die Stelle der Reste R¹³, R¹⁴, R¹⁵ bzw. R¹⁶ die Reste R²³, R²⁴, R²⁵ bzw. R²⁶ treten, die unabhängig voneinander C₁-C₈-Alkyl oder Benzyl bedeuten und einer der Reste R²³ bis R²⁶ auch Phenyl sein kann.

7. Verfahren nach Anspruch 1, dadurch gekennzeichnet, daß X die Bedeutung Fluorid, Chlorid, Bromid, Iodid, Sulfat, Nitrat, Phosphat, Formiat, Acetat, Propionat, Oxalat, Butyrat, Citrat, Succinat, Fumarat, Maleinat, Benzoat, Phthalat oder Laurinat, bevorzugt die Bedeutung Chlorid, Bromid, Iodid, Acetat, Laurinat, Nitrat oder Sulfat annimmt.

8. Verfahren nach Anspruch 1, dadurch gekennzeichnet, daß als aliphatische Monohydroxylverbindungen geradkettiges oder verzweigtes C₁-C₄-Alkanol, bevorzugt Methanol oder Ethanol eingesetzt wird.

9. Verfahren nach Anspruch 1, dadurch gekennzeichnet, daß der bifunktionelle Katalysator in einer Menge von 0,005 - 10 Gew.-%, bevorzugt 0,01 - 5 Gew.-%, besonders bevorzugt 0,02 - 3 Gew.-%, bezogen auf das vorlegte Alkylencarbonat, in der ersten Stufe und in einer Menge von 0,005 - 5 Gew.-%, bevorzugt 0,01 - 3 Gew.-%, besonders bevorzugt 0,01 - 1 Gew.-%, bezogen auf das gesamte Umesterungsgemisch, in der zweiten Stufe eingesetzt wird.

10. Verfahren nach Anspruch 1, dadurch gekennzeichnet, daß der nach der Aufarbeitung des ausreagierten Umesterungsgemisches im Rückstand verbliebene bifunktionelle Katalysator erneut in die erste Reaktionsstufe eingesetzt wird.

## Claims

1. Process for the preparation of dialkyl carbonates from alkylene oxides, CO₂ and alkanols in the presence of a catalyst, characterised in that, in a first step, an alkylene oxide having 2-8 C atoms is reacted with CO₂ in the alkylene carbonate which is to be formed first as reaction medium, at 40-190°C, preferably at 50-170°C and particularly preferentially at 60-160°C and a pressure below 10 bar, preferably below 8 bar, particularly preferentially below 5 bar and very particularly preferentially below 3 bar, and, in a second step, the alkylene carbonate thus formed is transesterified with a (cyclo)aliphatic C₁-C₁₀ monohydroxy compound, which can be substituted by C₃-C₆ cycloalkyl or phenyl, at 50-160°C, preferably at 60-150°C and particularly preferentially at 70-140°C, and under the autogenous pressure, both steps being carried out in the presence of a bifunctional catalyst of the formula
[Aₐ-X_{b}]ₘ . [B_{c}Y_{d}]ₙ (I)
wherein
A is the cation of a metal which belongs to the
third period and Group IIa, the
fourth period and Group IIa, IVa - VIIIa, Ib or IIb, the
fifth period and Group IIa, IVa - VIIa, IIb or IVb, or the
sixth period and Group IIa - VIa of the Periodic System of the elements in the short period form,
X is the anion of an inorganic or organic acid,
B represents a cation from the group comprising the alkali metal or alkaline earth metal cations, the quaternary ammonium, phosphonium, arsonium or stibonium ions and the ternary sulphonium cations,
Y is a halide ion, preferably bromide or iodide, particularly preferentially iodide, it being possible for X and Y to change positions if at least one of the anions is bromide or iodide,
a and b independently of one another represent integers from 1 to 5 and
c and d independently of one another represent integers from 1 to 3, the requirements in respect of the valencies of the cations and anions for the formation of a neutral salt having to be met, and
m and n independently of one another assume values of 0.001 - 1.

2. Process according to Claim 1, characterised in that an alkylene oxide of the formula 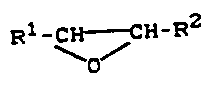 is used, wherein
R¹ denotes hydrogen, straight-chain or branched C₁-C₆ alkyl, straight-chain or branched C₂-C₆ alkenyl or chlorine and
R² denotes hydrogen or C₁-C₃ alkyl,
it being possible for R¹ and R² together also to represent trimethylene, tetramethylene or pentamethylene and the total number of C atoms not exceeding 8.

3. Process according to Claim 2, characterised in that the alkylene oxide used is ethylene oxide, propylene oxide, 1,2- or 2,3-butylene oxide, cyclohexene oxide, vinyloxirane or epichlorohydrin, preferably ethylene oxide or propylene oxide.

4. Process according to Claim 1, characterised in that A is the cation of one of the metals Mg, Ca, Sr, Ba, Zn, Cu, Mn, Co, Ni, Fe, Cr, Mo, W, Ti, Zr, Sn, Hf, V and Ta, preferably one of the metals Mg, Ca, Zn, Co, Ni, Mn, Cu and Sn.

5. Process according to Claim 1, characterised in that B is a cation of the formula 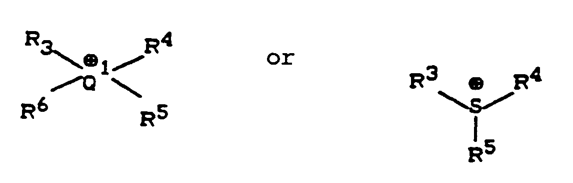 wherein
Q¹ represents N, P, As or Sb and
R³, R⁴, R⁵ and R⁶ independently of one another are straight-chain or branched C₁-C₁₈ alkyl or C₇-C₁₅ aralkyl and one of the radicals R³ to R⁶ can also be C₆-C₁₂ aryl,
and in that B is preferably a cation of the formula 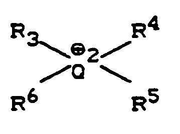 wherein
Q² represents N or P, preferably N.

6. Process according to Claim 5, characterised in that the radicals R¹³, R¹⁴, R¹⁵ and R¹⁶, which independently of one another denote straight-chain or branched C₁-C₁₂ alkyl or C₇-C₈ aralkyl, are present in place of the radicals R³, R⁴, R⁵ and R⁶ respectively and one of the radicals R¹³-R¹⁶ can also be phenyl, and in that, preferably, the radicals R²³, R²⁴, R²⁵ and R²⁶, which independently of one another denote C₁-C₈ alkyl or benzyl, are present in place of the radicals R¹³, R¹⁴, R¹⁵ and R¹⁶ respectively and one of the radicals R²³ to R²⁶ can also be phenyl.

7. Process according to Claim 1, characterised in that X assumes the meaning fluoride, chloride, bromide, iodide, sulphate, nitrate, phosphate, formate, acetate, propionate, oxalate, butyrate, citrate, succinate, fumarate, maleate, benzoate, phthalate or laurate and preferably assumes the meaning chloride, bromide, iodide, acetate, laurate, nitrate or sulphate.

8. Process according to Claim 1, characterised in that the aliphatic monohydroxy compounds used are straight-chain or branched C₁-C₄-alkanol, preferably methanol or ethanol.

9. Process according to Claim 1, characterised in that the bifunctional catalyst is used in the first step in an amount of 0.005 - 10 % by weight, preferably 0.01 - 5 % by weight and particularly preferentially 0.02 - 3 % by weight, based on the alkylene carbonate initially introduced, and in the second step in an amount of 0.005 - 5 % by weight, preferably 0.01 - 3 % by weight and particularly preferentially 0.01 - 1 % by weight, based on the total transesterification mixture.

10. Process according to Claim 1, characterised in that the bifunctional catalyst remaining in the residue after working up of the transesterification mixture which has reacted to completion is re-used in the first reaction step.

## Revendications

1. Procédé pour la préparation de dialkylcarbonates à partir d'oxydes d'alkylènes, de CO₂ et d'alcanols en présence d'un catalyseur, caractérisé en ce que, dans une première étape, on fait réagir un oxyde d'alkylène contenant 2 à 8 atomes de carbone avec du CO₂ dans l'alkylène-carbonate qui doit d'abord être formé, comme milieu réactionnel, à une température de 40-190°C, de préférence de 50-170°C, de manière particulièrement préférée de 60-160°C et sous une pression inférieure à 10 bar, de préférence inférieure a 8 bar, de manière particulièrement préférée inférieure a 5 bar, de manière tout particulièrement préférée inférieure à 3 bar et, dans une seconde étape, on procède à la transestérification de l'alkylène-carbonate ainsi formé avec un composé monohydroxylé (cyclo)aliphatique en C₁-C₁₀ qui peut être substitué par un groupe cycloalkyle en C₃-C₆ ou par un groupe phényle, à une température de 50-160°C, de préférence de 60-150°C, de manière particulièrement préférée de 70-140°C et sous la pression qui lui appartient en propre, dans lequel, dans les deux étapes, on travaille en présence d'un catalyseur bifonctionnel répondant à la formule
[Aₐ-X_{b}]ₘ . [B_{c}Y_{d}]ₙ
dans laquelle
A est le cation d'un métal qui appartient
à la troisième période et au groupe IIa,
à la quatrième période et au groupe IIa, IVa-VIIIa, Ib ou IIb,
à la cinquième période et au groupe IIa, IVa-VIIa, IIb ou IVb, respectivement
à la sixième période et au groupe IIa-VIa du système périodique des éléments dans la forme de courte période,
X représente l'anion d'un acide inorganique ou organique,
B représente un cation du groupe des cations de métaux alcalins ou alcalino-terreux, des cations d'ammonium, de phosphonium, d'arsonium ou de stibonium quaternaires et les cations de sulfonium ternaires,
Y représente un ion halogénure, de préférence bromure ou iodure, de manière particulièrement préférée iodure, X et Y pouvant échanger leur position lorsqu'au moins un des anions représente le bromure ou l'iodure,
a et b représentent, indépendamment l'un de l'autre, des entiers de 1 à 5, et
c et d représentent, indépendamment l'un de l'autre, des entiers de 1 à 3, les exigences quant aux valences des cations et des anions pour la formation d'un sel neutre devant être respectées,
m et n prennent indépendamment l'un de l'autre des valeurs de 0,001 à 1.

2. Procédé selon la revendication 1, caractérisé en ce qu'on met en oeuvre un oxyde d'alkylène de formule 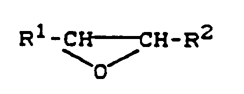 dans laquelle
R¹ représente un atome d'hydrogène, un groupe alkyle en C₁-C₆ à chaîne droite ou ramifiée, un groupe alcényle en C₂-C₆ à chaîne droite ou ramifiée ou encore un atome de chlore, et
R² représente un atome d'hydrogène ou un groupe alkyle en C₁-C₃,
R¹ et R² pouvant également représenter ensemble un groupe triméthylène, un groupe tétraméthylène ou un groupe pentaméthylène, le nombre total des atomes de carbone ne dépassant pas 8.

3. Procédé selon la revendication 2, caractérisé en ce qu'on met en oeuvre, comme oxyde d'alkylène, l'oxyde d'éthylène, l'oxyde de propylène, l'oxyde de 1,2- ou 2,3-butylène, l'oxyde de cyclohexène, le vinyloxiranne ou l'épichlorhydrine, de préférence l'oxyde d'éthylène ou l'oxyde de propylène.

4. Procédé selon la revendication 1, caractérisé en ce que A représente le cation d'un des métaux Mg, Ca, Sr, Ba, Zn, Cu, Mn, Co, Ni, Fe, Cr, Mo, W, Ti, Zr, Sn, Hf, V et Ta, de préférence un des métaux Mg, Ca, Zn, Co, Ni, Mn, Cu et Sn.

5. Procédé selon la revendication 1, caractérisé en ce que B représente un cation répondant aux formules 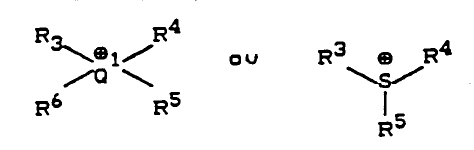 dans lesquelles
Q¹ représente N, P, As ou Sb, et
R³, R⁴, R⁵ et R⁶ représentent, indépendamment l'un de l'autre, un groupe alkyle en C₁-C₁₈ à chaîne droite ou ramifiée ou un groupe aralkyle en C₇-C₁₅, un des radicaux R³ à R⁶ pouvant également représenter un groupe aryle en C₆-C₁₂,
en ce que B représente de préférence un cation de formule 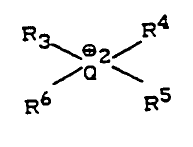 dans laquelle
Q² représente N ou P, de préférence N.

6. Procédé selon la revendication 5, caractérisé en ce que, prennent la place de R³, R⁴, R⁵, respectivement R⁶, les radicaux R¹³, R¹⁴, R¹⁵, respectivement R¹⁶ qui représentent, indépendamment l'un de l'autre, un groupe alkyle en C₁-C₁₂ à chaîne droite ou ramifiée ou un groupe aralkyle en C₇-C₈, un des radicaux R¹³ - R¹⁶ pouvant également représenter un groupe phényle, en ce que, de préférence prennent la place des radicaux R¹³, R¹⁴, R¹⁵, respectivement R¹⁶ les radicaux R²³, R²⁴, R²⁵, respectivement R²⁶ qui représentent, indépendamment l'un de l'autre, un groupe alkyle en C₁-C₈ ou un groupe benzyle, un des radicaux R²³ à R²⁶ pouvant également représenter un groupe phényle.

7. Procédé selon la revendication 1, caractérisé en ce que X prend la signification d'un fluorure, d'un chlorure, d'un bromure, d'un iodure, d'un sulfate, d'un nitrate, d'un phosphate, d'un formiate, d'un acétate, d'un propionate, d'un oxalate, d'un butyrate, d'un citrate, d'un succinate, d'un fumarate, d'un maléate, d'un benzoate, d'un phtalate ou d'un laurate, de préférence la signification d'un chlorure, d'un bromure, d'un iodure, d'un acétate, d'un laurate, d'un nitrate ou d'un sulfate.

8. Procédé selon la revendication 1, caractérisé en ce qu'on met en oeuvre, comme composés monohydroxylés aliphatiques, un alcanol en C₁-C₄ à chaîne droite ou ramifiée, de préférence le méthanol ou l'éthanol.

9. Procédé selon la revendication 1, caractérisé en ce qu'on met en oeuvre le catalyseur bifonctionnel en une quantité de 0,005-10 % en poids, de préférence de 0,01-5% en poids, de manière particulièrement préférée de 0,02-3% en poids, rapportés à l'alkylène-carbonate déposé au préalable, dans la première étape, et en une quantité de 0,005-5% en poids, de préférence de 0,01-3% en poids, de manière particulièrement préférée de 0,01-1% en poids, rapportés au mélange total de transestérification, dans la seconde étape.

10. Procédé selon la revendication 1, caractérisé en ce que, après le traitement du mélange de transestérification ayant complètement réagi, on remet en oeuvre, dans la première étape réactionnelle, le catalyseur bifonctionnel subsistant dans le résidu.
